# EUROPEAN PATENT APPLICATION

(11) **EP 3 800 250 A1**
(43) Date of publication of application: **07.04.2021**
(21) Application number: 19870253.2
(22) Date of filing: 10.10.2019
(51) Int. Cl.: C12N 15/06, C12N 5/071, C12N 5/22, C12N 15/07

(54) **METHOD FOR PRODUCING ANIMAL CELL CONTAINING DNA OF INTEREST USING MICRONUCLEATE CELL FUSION METHOD**

(30) Priority: 10.10.2018 JP 2018191994
(71) Applicant: National University Corporation Tottori University, Tottori-shi, Tottori 680-8550 (JP); Trans Chromosomics, Inc., Yonago-shi, Tottori 683-8503 (JP)
(72) Inventor: KAZUKI Yasuhiro, Yonago-shi, Tottori 683-8503 (JP); UNO Narumi, Yonago-shi, Tottori 683-8503 (JP); OSHIMURA Mitsuo, Tottori 683-8503 (JP)
(74) Representative: D Young & Co LLP
(86) International application number: PCT/JP2019/040091
(87) International publication number: WO 2020/075823

(57) **Abstract**

This application provides a method for preparing human cell-derived microcells from human cells comprising DNA of interest, comprising: a step of culturing human cells in a medium containing at least one micronucleus inducer selected from the group consisting of microtubule polymerization inhibitors (excepting colcemid), microtubule depolymerization inhibitors, and spindle checkpoint inhibitors, thereby to produce human cell-derived microcells; and a step of collecting human cell-derived microcells comprising the DNA of interest, and a method for preparing human cells comprising DNA of interest by use of the method, which can be also applied to non-human animal cells.

## Description

### TECHNICAL FIELD

The present invention relates to a method for producing a human cell-derived microcell that comprises DNA of interest from a human cell comprising DNA of interest, in good yield.

The present invention also relates to a method for producing a non-human animal cell-derived microcell that comprises DNA of interest from a non-human animal cell (for example, a rodent cell) comprising DNA of interest, in good yield.

The present invention further relates to a method for producing a human cell or non-human animal cell that comprises DNA of interest by fusing a human cell-derived microcell comprising DNA of interest, or a non-human animal cell (for example, a rodent cell)-derived microcell that comprises DNA of interest, serving as a donor cell and prepared by the above method of the present invention, with a human cell or a non-human animal cell, respectively, serving as a recipient cell.

### BACKGROUND ART

Microcell-mediated chromosome transfer (MMCT) is a technique for fusing a microcell prepared from a donor cell with a recipient cell. By this technique, a particular (foreign) DNA (for example, chromosome) in the donor cell can be transferred into the recipient cell. The microcell can be prepared by treating the donor cell usually with colcemid (Non Patent Literatures 1 to 3).

Conventionally, in the case where a human cell is used as a chromosome donor, a technique has been applied which comprises: introducing a drug resistant gene into a human cell; fusing the human cell with a mouse-derived A9 cell or a hamster-derived CHO cell capable of micronucleation to form a A9 hybrid cell or a CHO hybrid cell; treating the hybrid cell with colcemid to form micronuclei; and purifying microcells by drug selection. In contrast, when a microcell, which is purified from a human cell used as a donor cell, is fused with a human cell line (for example, human iPS cell), it is difficult to easily obtain a human cell line having a desired chromosome transferred therein by conventional MMCT. For the reason, A9 hybrid cell or CHO hybrid cell have been used as a donor cell.

A chromosome can be transferred into a recipient cell by MMCT. However, a vector for transferring a mega base (Mb)-size chromosome (for example, human chromosome or chromosome fragment) into a cell is limited. For example, a plasmid vector is limited to transfer of a polynucleotide of about 20 kb or less in size; the virus vector of a polynucleotide of 150 kb or less in size; BAC/PAC of a polynucleotide of 300 kb or less in size; and YAC of a polynucleotide of less than about 1 Mb. In contrast, in the case of an artificial chromosome such as a human artificial chromosome or a mouse artificial chromosome, the size of chromosome to be transferred is not particularly limited (Non Patent Literature 4). This allows a chromosome fragment comprising a desired locus to be integrated into such an artificial chromosome.

The human artificial chromosome and mouse artificial chromosome were developed for the first time by the present inventors and artificial chromosomes are called differently depending on their origins. More specifically, an artificial chromosome derived from a human chromosome is called a human artificial chromosome, whereas an artificial chromosome derived from a mouse is called a mouse artificial chromosome (Patent Literatures 1, 2 and 3).

Induced pluripotent stem (iPS) cell is a stem cell artificially prepared from a somatic cell by Dr. Shinya Yamanaka (Kyoto University) and has analogous features to those of embryonic stem (ES) cell, such as infinite proliferation and pluripotency (Non Patent Literatures 5 and 6). Since the iPS cell can be differentiated into somatic cells (stem cells, progenitor cells or mature cells) of various types of tissues, application of the iPS cells to regenerative medicine is accelerated. Since the iPS cells induced from a somatic cell of patients with hereditary diseases can be used for forming disease model cells, the iPS cells are used for drug development (Non Patent Literature 7).

If DNA of interest (for example, a chromosome, an artificial chromosome) can be easily transferred from a human cell (donor cell) to a human cell (recipient cell, for example, human iPS cell), great contribution to regenerative medicine and drug development is expected.

### PRIOR ART LITERATURE

### PATENT LITERATURE

Patent Literatures 1: JP Patent No. 4997544
Patent Literatures 2: JP Patent No. 5557217
Patent Literatures 3: JP Patent No. 4895100

### NON PATENT LITERATURE

Non Patent Literature 1: M. Katoh et al., BMC Biotechnology 2010, 10: 37
Non Patent Literature 2: N. Uno et al., Cytotechnology 2013, 65: 803-809
Non Patent Literature 3: M Hiratsuka et al., BMC Biotechnology 2015, 15: 58
Non Patent Literature 4: P. Osten et al., Handb Exp Pharmacol 2007, 178: 177-202
Non Patent Literature 5: K. Takahashi and S. Yamanaka, Cell 2006, 126 (4): 663-676
Non Patent Literature 6: K. Takahashi et al., Cell 2007, 131 (5): 861-872
Non Patent Literature 7: V.K. Singh et al., Frontiers in Cell and Developmental Biology 2015, doi: 10.3389/fcell.2015.00002

### SUMMARY OF INVENTION

### TECHNICAL PROBLEM

An object of the present invention is to provide a method for easily introducing DNA of interest (for example, a chromosome, an artificial chromosome) from a human cell (donor cell) to a human cell (recipient cell, for example, human iPS cell) or from a non-human animal cell (donor cell) to non-human animal cell (recipient cell, for example, iPS cell) by MMCT. As to MMCT, there has been no method for preparing a human cell-derived microcell directly from a human cell (donor cell). In a method conventionally employed, a mouse-derived A9 hybrid cell or a CHO hybrid cell is prepared and then fused with a human cell (recipient cell), as described above. In the conventional method, there are risks such as incorporation (contamination) of heterogeneous cytoplasm of an intermediate host, i.e., a CHO hybrid cell and a A9 hybrid cell, and viral infection.

To enable the aforementioned method, another object of the present invention is to provide a method for preparing a human cell-derived microcell from a human cell comprising DNA of interest in good yield. This method can be also used for efficiently preparing a non-human animal cell-derived microcell from a non-human animal cell comprising DNA of interest.

### SOLUTION TO PROBLEM

The present invention comprises the following features:
(1) A method for preparing human cell-derived microcells from human cells (excepting cancer cells) comprising DNA of interest, comprising culturing the human cells in a medium containing at least one micronucleus inducer selected from the group consisting of microtubule polymerization inhibitors (excepting colcemid), microtubule depolymerization inhibitors, and spindle checkpoint inhibitors, thereby to produce human cell-derived microcells, and collecting the human cell-derived microcells comprising DNA of interest.
(2) The method according to (1), wherein the DNA of interest is a normal human chromosome.
(3) The method according to (1), wherein the DNA of interest is a human artificial chromosome or mammalian artificial chromosome comprising a gene of interest.
(4) The method according to any one of (1) to (3), wherein the microtubule polymerization inhibitor is a mixture of colchicine and vincristine.
(5) The method according to any one of (1) to (3), wherein the microtubule depolymerization inhibitor is paclitaxel, docetaxel or a mixture of paclitaxel and docetaxel.
(6) The method according to any one of (1) to (3), wherein the spindle checkpoint inhibitor is reversine, hesperadin, or a mixture of reversine and hesperadin.
(7) A method for preparing non-human animal cell-derived microcells from non-human animal cells (excepting cancer cells) comprising DNA of interest, comprising culturing the non-human animal cells in a medium containing at least one micronucleus inducer selected from the group consisting of reversine, paclitaxel, or a mixture of reversine and paclitaxel, and a mixture of colchicine and vincristine, thereby to produce non-human animal cell-derived microcells, and collecting the non-human animal cell-derived microcells comprising the DNA of interest.
(8) The method according to (7), wherein the non-human animal cell is a rodent cell.
(9) The method according to (8), wherein the rodent cell is a Chinese hamster ovary (CHO) cell or a mouse A9 cell.
(10) The method according to any one of (7) to (9), wherein the DNA of interest is a normal human chromosome.
(11) T The method according to any one of (7) to (9), wherein the DNA of interest is a human artificial chromosome or a mammalian artificial chromosome, which comprises a gene of interest.
(12) A method for preparing human cells or non-human animal cells comprising DNA of interest, comprising: a step of preparing human cell-derived microcells comprising DNA of interest by the method according to any one of (1) to (6), or a step of preparing non-human animal cell-derived microcells comprising DNA of interest by the method according to any one of (7) to (11); a step of fusing the human cell-derived microcells or the non-human animal cell-derived microcells, which serve as donor cell, with human cells or non-human animal cells, respectively, serving as recipient cell; and a step of collecting the human cells or non-human animal cells comprising the DNA of interest.
(13) The method according to (12), wherein the non-human animal cell is a rodent cell.
(14) The method according to (13), wherein the rodent cell is a Chinese hamster ovary (CHO) cell or a mouse A9 cell.
(15) The method according to (12), wherein the DNA of interest is a normal human chromosome.
(16) The method according to (12), wherein the DNA of interest is a human artificial chromosome or mammalian artificial chromosome comprising a gene of interest.

The specification incorporates the contents of disclosure of JP Patent Application No. 2018-191994, based on which the priority right of the present application is claimed.

The present invention makes it possible to prepare a human cell-derived microcell or non-human animal cell-derived microcell directly from a human cell or a non-human animal cell (donor cell), and to fuse the microcell with a human cell or a non-human animal cell (recipient cell) by modified MMCT. Owing to this, if an artificial chromosome contains, for example, a disease-causing gene or a therapeutic gene, it is advantageous in that for example, a human cell such as a human iPS cell can be used for drug development and regenerative medicine.

### BRIEF DESCRIPTION OF DRAWINGS

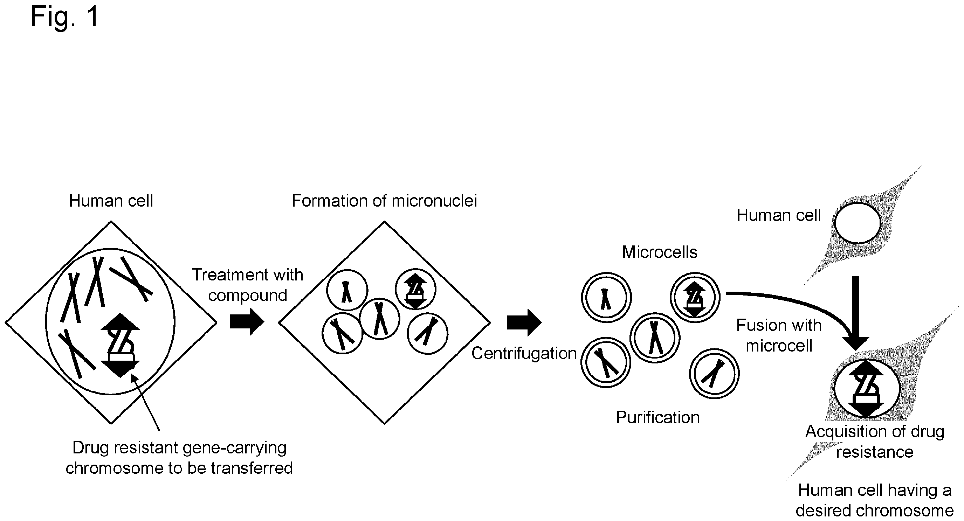

[Fig. 1] The figure shows an operation procedure of a method for introducing a chromosome or a chromosome fragment from, for example, a human cell serving as a donor cell, by microcell-mediated chromosome transfer (MMCT). The desired chromosome or chromosome fragment shown in the figure has a drug resistant gene. When the human cell is treated with a compound, formation of micronuclei is induced. The micronuclei are centrifuged. In this manner, microcells are prepared. The microcells are further purified by filtration. A human cell serving as a recipient cell and the microcell are fused (microcell-mediated chromosome transfer). In this manner, a desired chromosome or chromosome fragment is transferred into the human cell. The human cell having the desired chromosome or chromosome fragment transferred therein may be screened by drug selection and obtained as a drug resistant cell line. The same procedure applies to non-human animal cells.

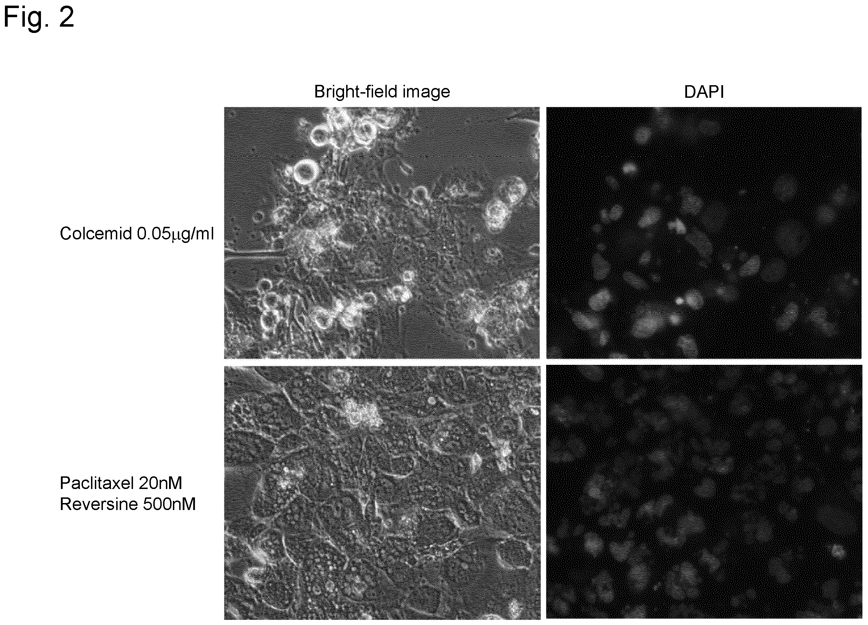

[Fig. 2] The figure shows typical examples of the micronucleation capability of human immortalized mesenchymal stem cells, which varies depending on the conditions of a compound or compounds used for treatment. The upper panels show the images of the cells treated with colcemid (0.05 µg/mL), whereas the lower panels show the images of the cells treated with paclitaxel (20 nM) and reversine (500 nM). The left panels show bright field images, whereas the right panels are images stained with DAPI.

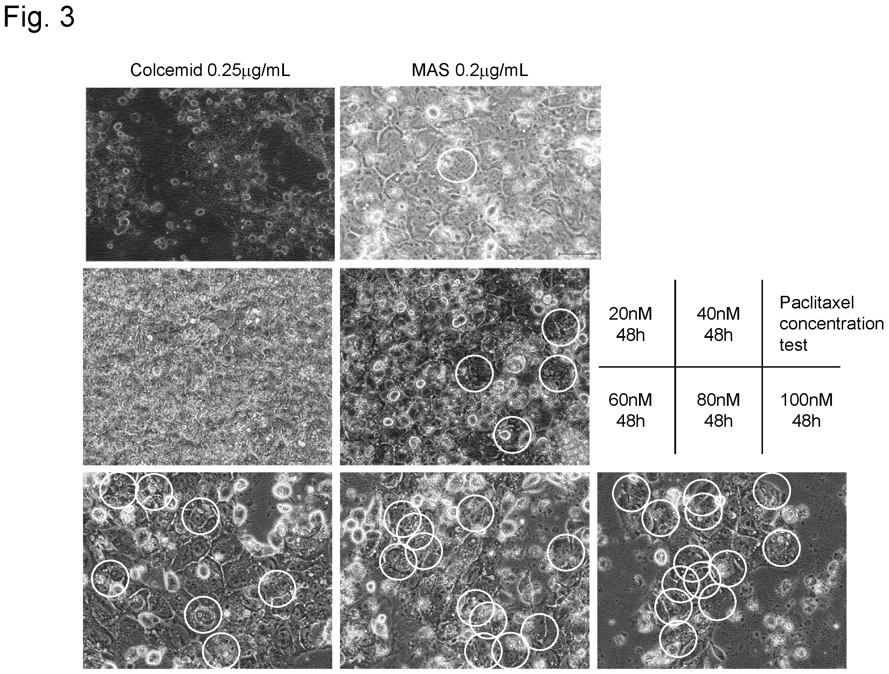

[Fig. 3] The figure shows typical examples of the micronucleation capability of human iPS cells, which varies depending on the conditions of a compound or compounds used for treatment. The upper left panel shows the image of cells treated with colcemid (0.25 µg/mL); whereas, the upper right panel shows the image of cells treated with 0.2 µg/mL MAS (a mixed reagent of colchicine and vincristine). The middle and lower panels show images of micronucleation, which varies depending on the concentration condition of paclitaxel. The cells having micronucleation are enclosed by circles.

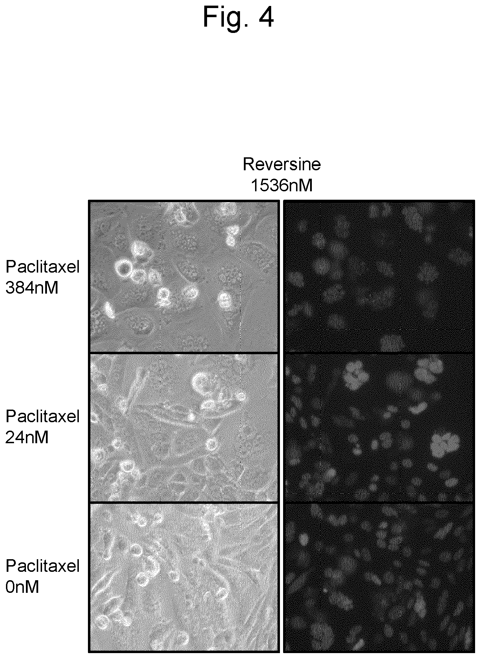

[Fig. 4] The figure shows typical examples of the micronucleation capability of CHO cells, which varies depending on the conditions of compounds used for treatment. The cells were treated with reversine (1536 nM), and then paclitaxel was added in a concentration: 384 nM, 24 nM or 0 nM. The left panels show bright field images, while the right panels show red fluorescence images for observation. The red fluorescent protein is localized within nuclei and morphology of nuclei can be identified.

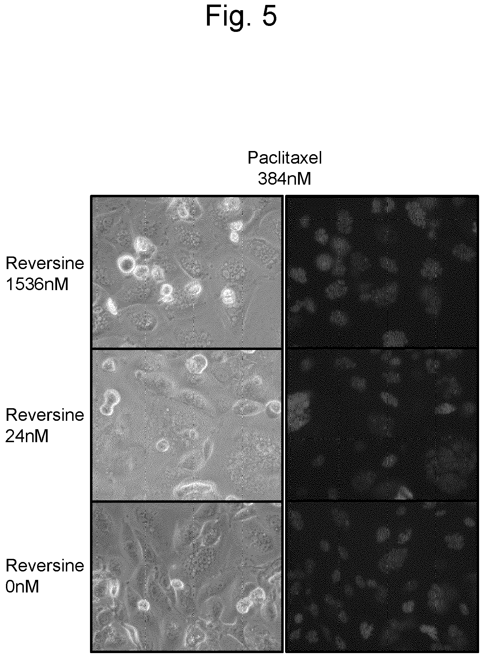

[Fig. 5] The figure shows typical examples of the micronucleation capability of CHO cells, which varies depending on the conditions of compounds used for treatment. The cells were treated with paclitaxel (384 nM), and then, treated by adding reversine in a concentration of 1536 nM, 24 nM or 0 nM. The left panels show bright field images; whereas, the right panels show red fluorescence images for observation. The red fluorescent protein is localized within nuclei and morphology of nuclei can be identified.

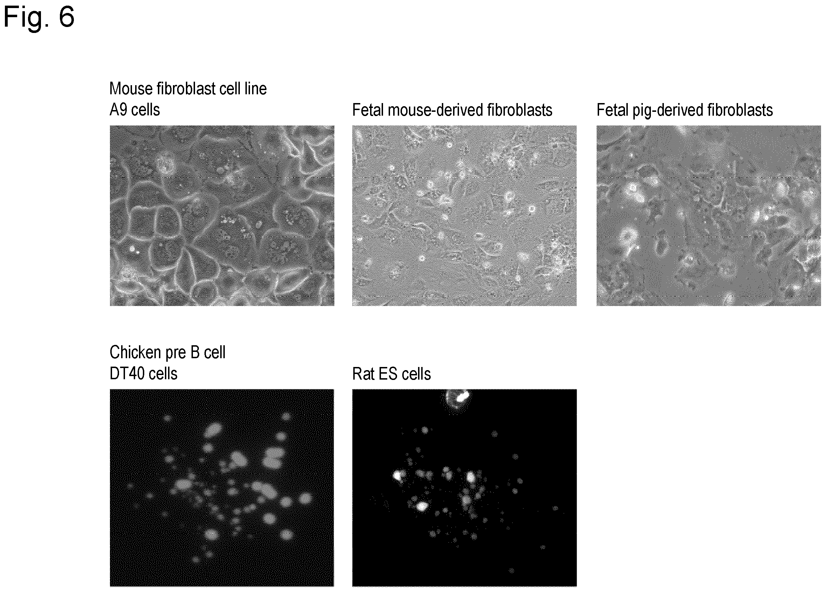

[Fig. 6] The figure shows typical examples of the micronucleation capability of mouse fibroblast cell line A9 cells, fetal mouse-derived fibroblast primary cultured cell line, fetal pig-derived fibroblast primary cultured cell line, chicken pre-B cell DT40 cells and rat ES cells, which varies depending on the conditions of compounds used for treatment: paclitaxel (4 to 400 nM) and reversine (0 to 1500 nM). The upper panels show typical microscopic images of the mouse fibroblast cell line A9 cell, fetal mouse-derived fibroblast primary cultured cell line and fetal pig-derived fibroblast primary cultured cell line. The lower panels show fluorescence microscopic images of chicken pre-B cell DT40 cells and rat ES cells stained with DAPI.

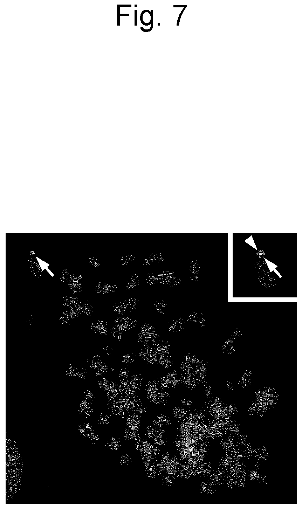

[Fig. 7] The figure shows an image of a human cell line, HT1080 (recipient cell) having a HAC vector transferred by microcell-mediated chromosome transfer from a human immortalized mesenchymal stem cell and analyzed by FISH. A HAC vector is co-stained in red and green. Centromere probes to human chromosomes 13 and 21 are stained in red (arrow) with p11-4. EGFP gene on the HAC vector is stained in green (arrowhead) by a pCAG-EGFP plasmid vector. The image of the HAC vector is magnified and shown in a small window.

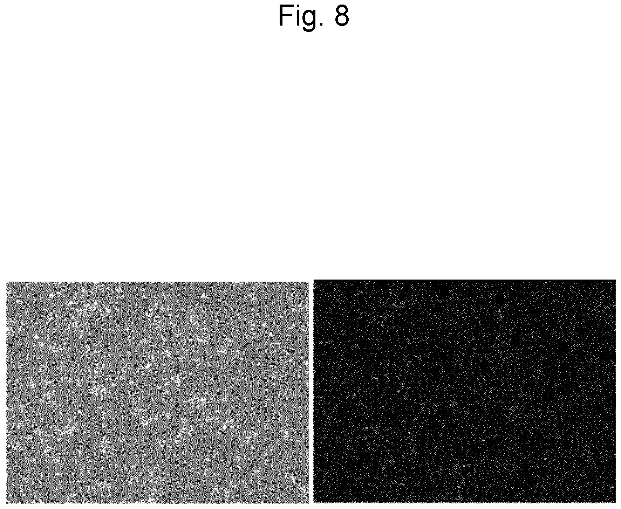

[Fig. 8] The figure shows fluorescence microscopic images of a human cell line, HT1080 (recipient cell) having a mouse artificial chromosome (MAC) vector transferred by microcell-mediated chromosome transfer from a human iPS cell (donor cell). The left panel shows a bright field image; whereas, the right panel shows a GFP fluorescence image.

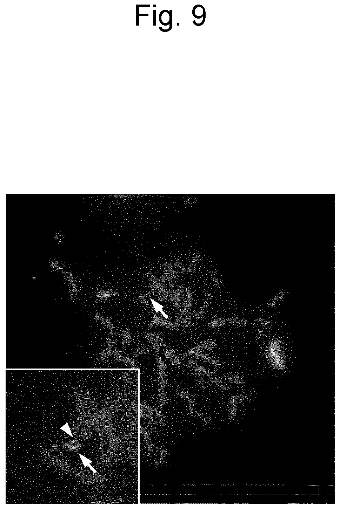

[Fig. 9] The figure shows an image of a human cell line, HT1080 (recipient cell) having a MAC vector transferred by microcell-mediated chromosome transfer from a human iPS cell (donor cell) and analyzed by FISH. MAC vector is stained in red (arrow) and green (arrowhead). The image of the MAC vector is magnified and shown in a small window.

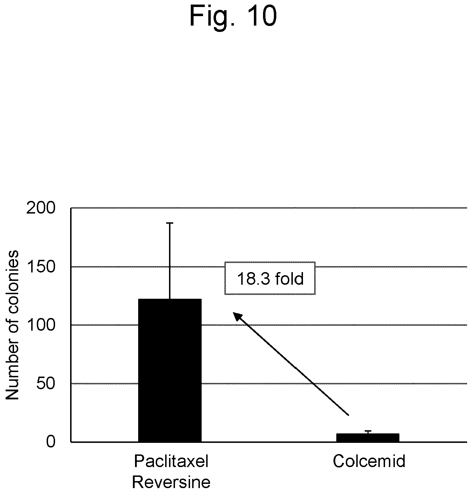

[Fig. 10] The figure shows the average number of drug-resistant colonies (average value and error range) when HAC vector was transferred from CHO cell (donor cell) into human cell line HT1080 (recipient cell) by microcell-mediated chromosome transfer in the experiment where the treatment with paclitaxel/reversine is compared to treatment with colcemid.

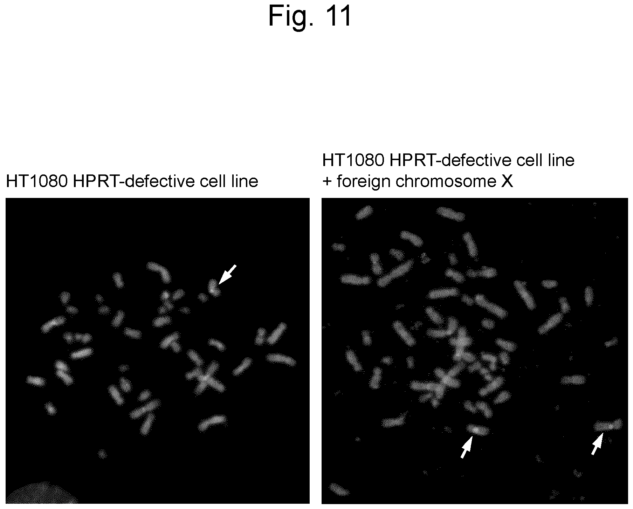

[Fig. 11] The figure shows an image of a human cell line, HT1080 HPRT defective cell line having an Chromosome X transferred from a human immortalized mesenchymal stem cell by microcell-mediated chromosome transfer and analyzed by FISH. The site stained in red (arrow) is the centromere of Chromosome X. It is stained in red by a human Chromosome X-specific alpha satellite probe. As a control experiment, an HT1080 HPRT defective strain having no Chromosome X transferred was analyzed. As a result, since the cell line is derived from a male, a single Chromosome X was observed (left panel). In contrast, in the Chromosome X-transferred cell line, two Chromosome Xs were observed (right panel).

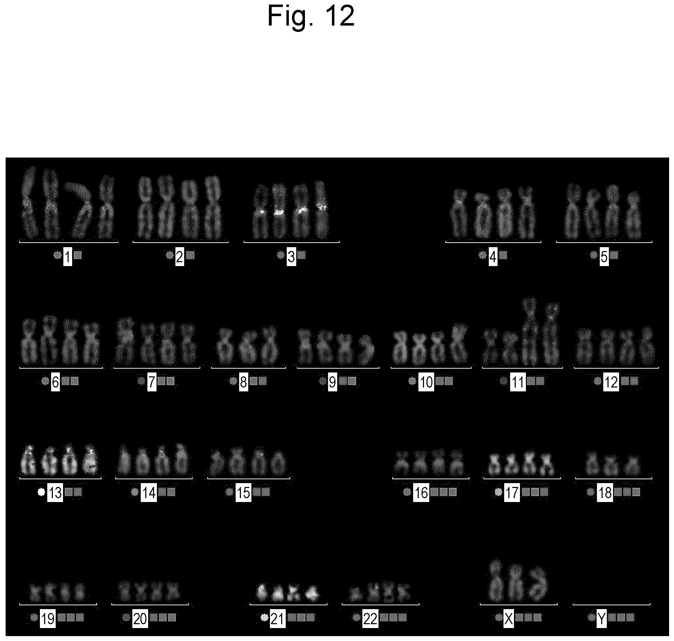

[Fig. 12] The figure show images of the human cell line, HT1080 HPRT defective cell line having an Chromosome X transferred from a human iPS cell by microcell-mediated chromosome transfer and analyzed by multicolor FISH analysis. Human chromosomes 1 to 22 and chromosomes X and Y were separately stained by use of color probes specific to individual chromosomes. Three Chromosome Xs labeled with blue were observed (left panel). Since different chromosomes other than Chromosome X were observed to have 4 copies excepting chromosomes 8, 18 and chromosome Y, the cell was found to be a tetraploid. When the parent cell line, HT1080 cell HPRT defective cell line is a tetraploid, it is known that two Chromosome Xs are present. This demonstrates that a human iPS cell-derived foreign Chromosome X was transferred into the HT1080 HPRT defective cell line, with the result that three Chromosome Xs were observed.

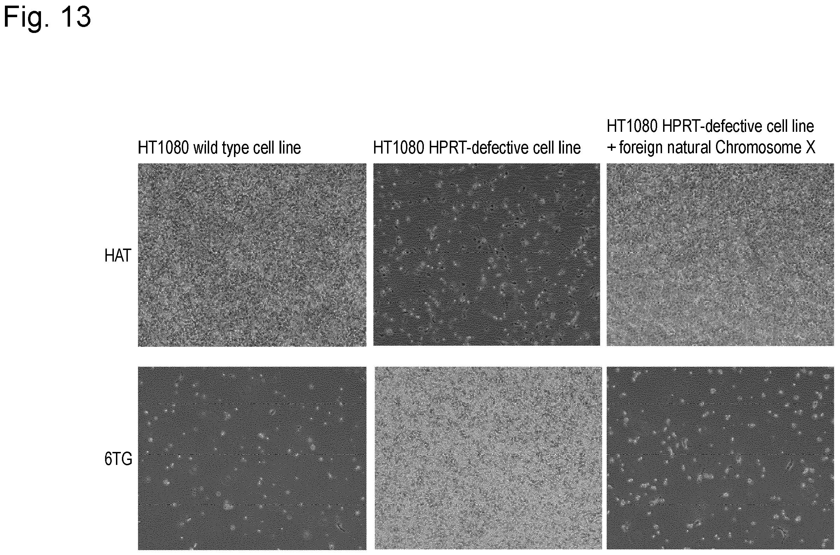

[Fig. 13] The figure shows the results of evaluation test on HAT and 6-thioguanine sensitivity of an HT1080 HPRT defective cell line having a Chromosome X (foreign chromosome) transferred from a human iPS cell. Microscopic images observed are shown when an HT1080 wild type cell line, an HT1080 HPRT defective cell line (middle row panel), and the foreign Chromosome X-comprising HT1080 HPRT defective cell line (right column panel) were screened by HAT (upper panel) and 20 µM 6-thioguanine (lower panel). The HT1080 wild type cell line, since it has a normal HPRT gene, was HAT resistant and 6-thioguanine sensitive (Fig. 13, left panel). Since HT1080 defective cell line, since it is defective in (internal) HPRT gene on the (internal) Chromosome X, was HAT sensitive and 6-thioguanine insensitive (middle panel). The HT1080 HPRT defective cell line comprising foreign Chromosome X, since it reacquired a HPRT gene, was HAT resistant and 6-thioguanine sensitive (Fig. 13, right panel) similarly to the wild type cell line.

### DESCRIPTION OF EMBODIMENTS

The present invention will be further specifically described.

### 1. Preparation of human cell-derived or non-human animal cell-derived microcell

According to a first embodiment of the present invention, there is provided a method for preparing human cell-derived microcells or non-human animal cell-derived microcells from human cells or non-human animal cells (excepting cancer cells) comprising DNA of interest, comprising a step of culturing the human cells or non-human animal cells comprising DNA of interest in a medium containing at least one micronucleus inducer selected from the group consisting of a microtubule polymerization inhibitor (excepting colcemid), a microtubule depolymerization inhibitor and a spindle checkpoint inhibitor to produce the human cell-derived microcells or the non-human animal cell-derived microcells; and a step of collecting the human cell-derived microcells or non-human animal cell-derived microcells comprising the DNA of interest.

The individual steps will be described below.

First of all, the human cell or non-human animal cell comprising DNA of interest will be described below.

The human cell or non-human animal cell is not particularly limited as long as it is not a cancer cell (also referred to as "a tumor cell"), which is a cell line derived from a cancer tissue; any other cell is included. Examples of the human cell or non-human animal cell include a cell and stem cell derived from an organ and a tissue of an organ, such as a primary cultured cell, an established cell, an immortalized cell, a cell deposited in ATCC and an artificial pluripotent stem (iPS) cell thereof. Non-limiting examples of the human cell or non-human animal cell include an epithelial cell, a fibroblast, an endothelial cell, a hepatocyte, a pancreatic cell, a kidney cell, a brain cell, a muscle cell, a chondrocyte, an osteoblast, a cardiomyocyte, a hair papilla cell, a nerve cell, a tissue stem cell, an embryonic stem cell and a hematopoietic stem cell.

Preferred non-limiting examples of the human cell or non-human animal cell are an iPS cell or an immortalized mesenchymal stem cell.

In the specification, the term "DNA of interest" refers to DNA having a distinctive purpose of use. In consideration of use of MMCT, for example, a human derived chromosome or a chromosome fragment and an artificial chromosome are included. Examples of the artificial chromosome include a human artificial chromosome and a mammalian artificial chromosome (for example, mouse artificial chromosome or rat artificial chromosome). The DNA of interest is preferably a normal human chromosome or may be a human artificial chromosome or mammalian artificial chromosome having a gene of interest.

In the specification, the term "gene of interest" refers to a useful gene having a distinctive purpose of use. In some cases, the gene may be contained in a locus or a chromosome fragment. Alternatively, a gene of interest may be transferred into an artificial chromosome as a foreign gene or may be previously integrated into an artificial chromosome.

Non-limiting examples of the gene of interest include a disease-causing gene, a therapeutic gene, chromosome fragments comprising these genes, and a gene necessary for cell differentiation. Examples thereof include genes or DNAs encoding proteins such as a cytokine, an interferon, an interleukin, a chemokine (a factor having cell migration property), a granulocyte colony stimulating factor, a tumor necrosis factor, a growth factor (for example, a platelet-derived growth factor, a vascular endothelial growth factor, a hepatocyte growth factor, a keratinocyte growth factor, a nerve growth factor), a nutritional factor (for example, neurotrophin, a brain-derived neurotrophic factor), erythropoietin, a blood coagulation protein, a platelet production promoter, a hormone, an antibody (for example, a monoclonal antibody, a recombinant antibody such as scFv) and an enzyme. Examples of the gene of interest further include therapeutic genes or DNAs involved in a disease such as muscular dystrophy, hemophilia, a neurodegenerative disease, an autoimmune disease, an allergic disease, a hereditary disease and a tumor; and an immune genes or DNAs such as a T cell receptor (TCR) and a human leukocyte antigen (HLA).

In the specification, the term "artificial chromosome" refers to a chromosome-derived vector artificially prepared, comprising a centromere, a long arm and, if exists, a fragment of a short arm near a centromere (genes and the like are removed as much as possible) and natural or artificial telomere of a chromosome derived from a mammalian animal (also referred to as "mammal") including a human and a rodent (for example, mouse, rat). Accordingly, the artificial chromosome of the present invention differs from a conventional vector such as a virus, YAC, BAC, PAC, a cosmid and a plasmid.

The artificial chromosome contains a site into which a desired nucleic acid such as a gene, a locus and a chromosome fragment, e.g., a human derived desired nucleic acid, is to be inserted. A desired nucleic acid is inserted into this site.

A mouse artificial chromosome is described, for example, in JP Patent No. 5557217 and JP Patent No. 4997544; whereas, a human artificial chromosome is described, for example, in JP Patent No. 4895100.

A mouse chromosome for use in preparation of a mouse artificial chromosome may be any one of mouse chromosomes 1 to 19, X and Y, and preferably, any one of chromosomes 1 to 19. For example, in the case of an artificial chromosome vector derived from a fragment of mouse chromosome 11, the long arm fragment consists of, for example, a long arm fragment obtained by removing a distal region from AL671968 or BX572640 of chromosome 11 (positioned on the side nearer to the centromere than AL671968), CR954170 (positioned on the side nearer to the centromere than AL671968 and BX572640) or AL713875 (positioned on the side nearer to the centromere than AL671968), but not limited to this. Alternatively, in the case of a mouse artificial chromosome derived from a mouse chromosome 15 fragment, the long arm fragment consists of a long arm fragment obtained by removing a distal region from a position such as AC121307 and AC161799. Alternatively, in the case of a mouse artificial chromosome derived from a mouse chromosome 16 fragment, the long arm fragment consists of a long arm fragment obtained by removing a distal region from a position such as AC127687 and AC140982.

The human chromosome for use in preparation of a human artificial chromosome may be any one of human chromosomes 1 to 22, X and Y; and preferably any one of chromosomes 1 to 22. Alternatively, as the human chromosome, a chromosome derived from a human iPS cell may be used. Preparation method for a human artificial chromosome is referred to those disclosed, for example, in JP Patent Publication (Kokai) No. 2010-004887A and WO2008/013067, and techniques for preparing a mouse artificial chromosome disclosed in the above literatures. Since the homologous recombination frequency of an iPS cell is high, a human artificial chromosome may be constructed by use of a human iPS cell-derived chromosome.

The artificial chromosome may further contain an DNA sequence insertion site for inserting a desired nucleic acid (for example, gene, locus, chromosome fragment), such as loxP (Cre recombinase recognition site), FRT (Flp recombinase recognition site), φC31attB and φC31attP (φC31 recombinase recognition site), R4attB and R4attP (R4 recombinase recognition site), TP901-1attB and TP901-1attP (TP901-1 recombinase recognition site) or Bxb1attB and Bxb1attP (Bxb1 recombinase recognition site). Since an artificial chromosome may contain a site for inserting a desired nucleic acid sequence, if the sequence of a desired nucleic acid is integrated into the site, the desired nucleic acid can be expressed when the artificial chromosome is transferred into a cell.

Non-limiting examples of the desired nucleic acid includes a disease-causing gene, a therapeutic gene, chromosome fragments comprising the respective genes, a reporter gene and a marker gene.

On the vicinity or both sides of the insertion site of a desired nucleic acid sequence in the artificial chromosome of the present invention, at least one insulator sequence is allowed to be present. The insulator sequence has an enhancer blocking effect (by which adjacent genes are not mutually influenced) or a chromosome boundary effect (isolating and distinguishing the region at which gene expression is ensured and the region at which gene expression is suppressed). Examples of such a sequence may include human β globins HS1 to HS5 and chicken β globin HS4.

A DNA of interest as mentioned above can be transferred into the donor cell, more specifically, into an artificial chromosome such as a human artificial chromosome or a mammalian artificial chromosome of the donor cell by a gene transfer technology such as a lipofection method. After gene introduction, the donor cell comprising the DNA of interest may be detected, for example, by the HAT selection method and collected.

In order to screen a desired microcell, a drug resistant gene (for example, blasticidin resistant gene, neomycin (G418) resistant gene) and/or a gene encoding a fluorescent protein (for example, GFP, DsRed) can be transferred into a human cell or a non-human animal cell.

Next, a micronucleus inducer and micronucleation will be described (Fig. 1).

The method of the present invention is characterized by culturing human cells or non-human animal cells comprising DNA of interest in a medium containing at least one micronucleus inducer selected from the group consisting of a microtubule polymerization inhibitor (excepting colcemid), a microtubule depolymerization inhibitor and a spindle checkpoint inhibitor.

The microtubule polymerization inhibitor is not particularly limited as long as it forms a microcell and colcemid is not included; for example, a mixture of colchicine and vincristine is included.

The microtubule depolymerization inhibitor is not particularly limited as long as it forms a microcell; for example, paclitaxel, docetaxel and a mixture of docetaxel and paclitaxel are included.

The spindle checkpoint inhibitor is not particularly limited as long as it forms a microcell; for example, reversine, hesperadin and a mixture of reversine and hesperadin are included.

A preferable concentration of a micronucleus inducer in the medium, which varies depending on the type of micronucleus inducer, is, for example, 0.001 nM to 1 M in the case of reversine, 0.001 nM to 1 M in the case of paclitaxel or 0.001 nM to 1 M in the case of a mixture of colchicine and vincristine (contained in a ratio of 0.01: 9.99 to 9.99: 0.01 (weight)); however, the preferable concentration is not limited to fall in the above range as long as a microcell is formed. A preferable micronucleus inducer is reversine or a mixture of reversine and paclitaxel.

The culture medium is not limited as long as it is used for culturing animal cells. Examples of a basic medium include Dulbecco modified Eagle's medium (DMEM), Eagle's minimum essential medium (EMEM), Ham F-12 culture medium, RPMI-1640 culture medium, Iscove's modified Dulbecco medium (IMDM) and a mixture of these. As a medium for a human iPS cell, primate ES cell medium (REPROCELL, Japan) may be used.

A method for inducing micronucleation may be carried out, for example, in the following procedure.

A human cell or non-human animal cell comprising DNA of interest is used as a donor cell. The donor cells are cultured in a cell-culture flask (for example, bottom area: 25 cm²). At about a 70% confluence, a micronucleus inducer is added to the aforementioned culture medium and culture is carried out in the presence of 20% fetal calf serum (FCS) at 37°C in the condition of 5% CO₂ for 12 to 96 hours or more.

Finally, a microcell population is collected by culturing as mentioned above, and further a human cell-derived microcell or non-human animal cell-derived microcell comprising DNA of interest is collected based on "fluorescence positive" and by drug selection.

The microcells may be collected, for example, in the following manner.

After the induction of micronucleation, the culture medium in the flask is removed by aspiration. The flask is filled up to a level of 90% with cytochalasin B. The flask is inserted into a specialized container for a large-size high-speed centrifuge (for example, BECKMAN) and hot water (34°C) is added so as not to completely cover the flask. Centrifugation (Rortor ID 10.500, 8,000 rpm, 1 hour, 34°C) is carried out. After completion of centrifugation, cytochalasin B is recovered, the pellet in the flasks are collected with 2 ml of serum-free medium DMEM in 15-ml tubes and slowly filtered by filters (in the order of 8 µm→5 µm→3 µm). Each tube is centrifuged (1,200 rpm, 5 minutes, room temperature). The supernatant is removed by aspiration, the pellets of the tubes are collected with 5-ml serum-free medium DMEM, suspended and centrifuged (2000 rpm, 5 minutes). In this manner, microcells are collected.

The method of the present invention can be also used for preparing a non-human animal cell-derived microcell from a non-human animal cell (excepting cancer cell) comprising DNA of interest by use of a micronucleus inducer as mentioned above. Because of this, the method is also included in the present invention.

More specifically, according to a second embodiment, there is provided a method for preparing non-human animal cell-derived microcells from non-human animal cells (excepting cancer cells) comprising DNA of interest, comprising culturing the non-human animal cells in a medium containing at least one micronucleus inducer selected from the group consisting of reversine, paclitaxel or a mixture of reversine and paclitaxel, and a mixture of colchicine and vincristine thereby to produce non-human animal cell-derived microcells; and collecting non-human animal cell-derived microcells comprising the DNA of interest.

The non-human animal cell refers to any of animal cells. Non-limiting examples thereof include rodent cells, Artiodactyla cells, Ungulate cells, primate (excepting a human) cells, canine cells, feline cells, avian cells, reptile cells, amphibian cells, and insect cells. The non-human animal cell is preferably a rodent cell.

Examples of the rodent cells include cells derived from organ or tissues of animals such as mouse, rat, and hamster, for example, primary cultured cells, established cells thereof, and cells deposited in ATCC. Non-limiting examples of the rodent cells include epithelial cells, fibroblast cells, endothelial cells, hepatocyte, muscle cells, chondrocyte, osteoblast, cardiomyocyte, hair papilla cells, nerve cells, and ovarian cells.

As the rodent cells, Chinese hamster ovary (CHO) cell and mouse derived A9 cell are preferable.

The same descriptions as in the human cells or non-human animal cells are applied to the DNA of interest and the micronucleus inducer herein.

### 2. Preparation of human cell or non-human animal cell comprising DNA of interest

According to a third embodiment of the present invention, there is provided a method for preparing human cells or non-human animal cells comprising DNA of interest, comprising: a step of preparing human cell-derived microcells from human cells comprising DNA of interest, or non-human animal cell-derived microcells from non-human animal cells (excepting cancer cells) comprising DNA of interest, by the method described in Section 1 above; a step of fusing the human cell-derived microcells or the non-human animal cell-derived microcells, with human cells or non-human animal cells, respectively; and a step of collecting human cells or non-human animal cells comprising the DNA of interest.

Now, the steps will be individually described below.

The step of cell fusion comprises co-culturing microcells comprising DNA of interest prepared in Section 1 above and a human cell line serving as recipient cell in the absence of feeder cells to fuse the cells by use of modified MMCT (microcell-mediated chromosome transfer), thereby preparing a human cell line comprising the DNA of interest.

Examples of the human cell or non-human animal cell include a normal cell, an immortalized cell, an embryonic stem cell, a somatic stem cell, and an iPS cell (for example, derived from human or mammalian somatic cell); however, the human cell or non-human animal cell is not limited as long as DNA of interest can be transferred into the cell.

The modified MMCT (microcell-mediated chromosome transfer) comprises expressing a viral envelope protein on the surface of a microcell. For that purpose, the human cell or non-human animal cell serving as a donor cell may be transformed in advance with DNA encoding the viral envelope protein.

The viral envelope protein refers to an envelope protein derived from a virus such as measles virus and leukemia virus. Examples of the envelope protein may include a measles virus-derived envelope protein such as a hemagglutinin (H) protein and a fusion (F) protein and a leukemia virus-derived envelope protein such as SU protein and TM protein. A preferable viral envelope protein is the measles virus-derived envelope protein such as H protein and F protein.

H protein may be modified by a genetic engineering technique such that H protein can be bound to a surface antigen of a predetermined recipient cell. For example, if a minimum unit of an antibody recognizing an antigen, single chain Fv (ScFv), preferably an anti-CD9 antibody, anti-CD13 antibody or anti-CD71 antibody or ScFv of each of these antibodies is fused with H protein, fusion can be made to a cell type with which a wild-type H protein cannot fuse. In order to provide a fusion ability, a recipient cell, i.e., a chromosome receiving cell, is allowed to temporarily express a CD46 antigen and/or a CD120 (SLAM) antigen that can bind to H protein. In this manner, a microcell fusion may be made. Alternatively, if a Tag peptide sequence can be added to H protein and a recipient cell is genetically modified such that an anti-His tag antibody or an anti-H protein antibody is expressed, fusion can be made.

A plasmid comprising DNA encoding an envelope protein as mentioned above or a plasmid comprising a DNA cassette encoding a plurality of envelope proteins is constructed by a genetic recombination technique, and a donor cell is transformed by any one of these plasmids. To avoid occurrence of autologous cell fusion when the transformed cell is produced, CD46 antigen and/or CD120 antigen of a donor cell or a surface antigen, which is to be bound to an antibody ScFv fused with H protein, are deleted. In this manner, microcell-mediated chromosome transfer may be attained.

Into a human cell or non-human animal cell serving as a donor cell, a drug resistant gene (for example, blasticidin resistant gene, neomycin (G418) resistant gene) and/or a gene encoding a fluorescent protein (for example, GFP, DsRed) may be further transferred in order to screen a desired cell.

In the aforementioned method, a donor cell that expresses a viral envelope protein on the cell surface and contains DNA of interest (for example, artificial chromosome) may be prepared.

If the recipient cell is a human cell or a non-human animal cell, DNA of interest (for example, an artificial chromosome such as a human artificial chromosome and a mammalian artificial chromosome) can be transferred into the human cell or non-human animal cell by a feeder-free culture method and MMCT using a viral envelope.

iPS cells are produced as a colony by introducing certain reprogramming factors (DNAs or proteins) into somatic cells (including, for example, somatic stem cells, progenitor cells and/or mature cells) and culturing and then subculturing the somatic cells in appropriate culture media for about 3 to 5 weeks. As the reprogramming factor, a combination of Oct3/4, Sox2, Klf4 and c-Myc; a combination of Oct3/4, Sox2 and Klf4; a combination of Oct4, Sox2, Nanog and Lin28; or a combination of Oct3/4, Sox2, Klf4, c-Myc, Nanog and Lin28, is known (K. Takahashi and S. Yamanaka, Cell; 126: 663-676 (2006); WO2007/069666; M. Nakagawa et al., Nat. Biotechnol. 26: 101-106 (2008); K. Takahashi et al., Cell; 131: 861-872 (2007); J. Yu et al., Science; 318: 1917-1920 (2007); J. Liao et al., Cell; Res. 18,600-603 (2008)). For culture, for example, mitomycin C-treated fetal mouse fibroblast cell line (for example, STO) is used as feeder cells. On the feeder cell layer, reprograming-factor expressing vector-transferred somatic cells (about 10⁴ to 10⁵ cells/cm²) are cultured by using ES cell culture medium and at a temperature of about 37°C. In this culture, the feeder cells herein are not always necessary (Takahashi, K. et al., Cell; 131: 861-872 (2007)). Examples of the basic medium include Dulbecco modified Eagle's medium (DMEM), Eagle's minimum essential medium (EMEM), Ham F-12 culture medium, RPMI-1640 culture medium, Iscove's modified Dulbecco medium (IMDM) and a mixture of these mediums. As the human iPS cell medium, e.g., a primate ES cell medium (REPROCELL) may be used.

Examples of the culture medium for iPS cells without a feeder cell include StemFit™ AK02N (REPROCELL), TeSR™ (STEMCELL Technologies) and NutriStem XF/FF Culture Medium (STEM GWNT). Examples of the culture substrate include Geltrrex™ LDEV-Free hESC-qualified (Thermo Fisher Scientific), iMatrix™-511 (Nippi. Inc.) and hES Qualified Matrigel™ (Corning).

Cell fusion is carried out by co-culturing human cell-derived microcells comprising DNA of interest, or non-human animal (for example, a rodent) cell-derived microcells comprising DNA of interest, and human cells, or non-human animal cells, serving as recipient cells prepared in Section 1 above, in a culture medium exemplified above without feeder cells. Thereafter, the fused cells are cultured in a medium containing, for example, a drug (for example, an antibiotic). Human cells or non-human animal cells comprising DNA of interest may be screened based on drug resistance (and, positive fluorescence) as an indicator.

The culture conditions for cell fusion are: for example, culture medium, DMEM containing 10% fetal calf serum (FCS); culture temperature, 37°C; and culture period, 24 hours. Alternative culture conditions are: for example, culture medium, Stemfit; substrate, culture dish coated with iMatrix-511 (0.5 µg/mL); culture temperature, 37°C; and culture period, 24 hours.

### EXAMPLES

The present invention will be more specifically described with reference to the following Examples; however, the scope of the present invention is not limited by Examples.

### <Example 1>

### Search of micronucleus inducer for animal cell

### [A] Search of micronucleus inducer for human immortalized mesenchymal stem cell

As a candidate micronucleus inducer, a cell division inhibitor is included. Examples thereof include a microtubule polymerization inhibitor, a microtubule depolymerization inhibitor (or microtubule stabilizer) and a M-phase checkpoint protein inhibitor. More specifically, colcemid and MAS (i.e., the mixed reagent of colchicine and vincristine) were used as the microtubule polymerization inhibitor, and paclitaxel was used as a microtubule depolymerization inhibitor. Micronucleation efficiencies of these agents were compared. In addition to these agents, reversine, which is an inhibitor of M-phase checkpoint proteins, i.e., aurora kinases A and B, was added. A synergetic effect by reversine was studied.

### [A.1.1] Seeding of test cell and treatment with various inhibitors

Human immortalized mesenchymal stem cells (hiMSC: provided by Dr. Toguchida, Kyoto University) were seeded in individual wells of a 6-well plate at a density of 5 × 10⁵ cells per well and cultured at 37°C in the condition of 5% CO₂ for 24 hours. For the micro nucleation induction experiment, 26 conditions in total were designed based on treatments with colcemid (0.05 µg/mL, 0.1 µg/mL, 0.2 µg/mL), MAS (0.1 µg/mL, 0.2 µg/mL, 0.4 µg/mL) and paclitaxel (0.8 nM, 4 nM, 20 nM, 100 nM, 400 nM), with the addition of reversine (no addition, 500 nM, 1000 nM, 1500 nM) during the treatments, and the cells were cultured at 37°C in the condition of 5% CO₂ for 48 hours.

### [A.1.2] Micronucleation efficiency observed by DAPI staining

In order to observe living cells after micronucleation induction, the cells were fixed with 4% paraformaldehyde and stained with DAPI (concentration: 1 µg/mL) for 15 minutes. The fixed cells were observed by fluorescence microscopy and the micronuclear formation induction rates were compared. Whether or not micronuclei were formed in individual cells was observed and the results are shown in a figure (Fig. 2). From the results, it was found that when the cells were treated with paclitaxel (20 nM) and reversine (500 nM), micronuclei were formed most efficiently. It was also found that not only a microtubule polymerization inhibitor such as colcemid conventionally used but also a microtubule depolymerization inhibitor such as paclitaxel was effective. It was further found that combination with the M-phase checkpoint inhibitor reversine is useful.

### [B] Search of micronucleus inducer for human iPS cells

### [B.1.1] Culture of human iPS cells

Human iPS cells were seeded to a 6-well plate previously coated with 0.5 µg/cm² Laminin™-511 (Nippi. Inc.) at a density of 5 × 10⁵ cells per well, and 10 µM Y-27632 was added. The human iPS cells were incubated at 37°C in the condition of 5% CO₂ for 24 hours. For a micronucleation induction experiment, 22 conditions in total were designed based on treatments with colcemid 0.25 µg/mL, MAS 0.2 µg/mL or paclitaxel (20 nM, 40 nM, 60 nM, 100 nM, 400 nM) with the addition of reversine (no addition, 500 nM, 1000 nM, 1500 nM) during the treatments, and the cells were cultured at 37°C in the condition of 5% CO₂ for 48 hours.

### [B.1.2] Micronucleation efficiency based on microscopic observation

After micronucleation induction, the micronucleation induction rates were compared based on microscopic observation. As a result, micronucleation induction was observed in MAS (in the condition of 0.2 µg/mL) compared to colcemid (in the condition of 0.25 µg/mL) (Fig. 3). Further, in the case of paclitaxel, it was observed that the micronucleation rate increases in a concentration dependent manner (Fig. 3). In addition, it was found that combination with the M-phase checkpoint inhibitor reversine is useful.

### [C] Verifying the effect of micronucleus inducer on CHO cell

### [C.1.1] Culture of CHO cell

CHO MHG#4a cells expressing a red fluorescent protein exhibiting intranuclear localization were seeded in the wells of a 96-well plate at a density of 1 × 10⁴ cells per well. Ninety six conditions, which were designed by combinations of paclitaxel (0 nM, 96 nM, 192 nM, 384 nM, 768 nM, 1152 nM, 1536 nM, 1920 nM) and reversine (0 nM, 192 nM, 384 nM, 768 nM, 1536 nM, 3072 nM, 4608 nM, 6144 nM, 7680 nM, 9216 nM, 10752 nM, 12288 nM), were assigned to the wells. Culture was carried out at 37°C in the condition of 5% CO₂ for 72 hours.

### [C.1.2] Micronucleation efficiency by fluorescence-microscopic observation

After micronucleation induction, the micronucleation induction rates were compared based on observation by fluorescence microscopy. Typical results are shown (Fig. 4 and Fig. 5). As a result, it was observed that the micronucleation rate increases in a concentration dependent manner in the cases of paclitaxel and reversine.

### [D] Verifying the effect of micronucleus inducer on non-human animal cells

Micronucleation induction by colcemid and, paclitaxel and reversine, in non-human animal-derived cells was checked. More specifically, mouse fibroblast cell line A9 cells, fetal mouse-derived fibroblast primary cultured cell line, chicken pre-B cell DT40 cells, rat ES cells, and fetal pig-derived fibroblasts were treated with colcemid and, paclitaxel and reversine, and the presence or absence of micronucleation was evaluated by microscopic observation.

### [D.1.1] Seeding of test cells and treatment with various inhibitors

Mouse fibroblast cell line A9 cells, a fetal mouse-derived fibroblast primary cultured cell line, fetal pig-derived fibroblasts, chicken pre-B cells, i.e. DT40 cells, and rat ES cells were seeded in wells of a 24-well plate at a density of 1 × 10⁵ cells per well and cultured at 37°C in the condition of 5% CO₂ for 24 hours. For a micronucleation induction experiment, treatments with paclitaxel (4 nM, 25 nM, 100 nM, 400 nM) were divided into two groups based on the addition or no addition of reversine (0 nM, 500 nM, 1000 nM, 1500 nM) during the treatments. Twenty conditions were thus designed in total. The cells were cultured at 37°C in the condition of 5% CO₂. At a time point of 24, 48 and 72 hours, microscopic observation was carried out.

### [D.1.2] Micronucleation efficiency by microscopic observation

The micronucleation induction rates were compared based on microscopic observation. Whether or not micronuclei were produced in the mouse fibroblast cell line A9 cells, fetal mouse-derived fibroblast primary cultured cell line, and fetal pig-derived fibroblasts was observed and bright field images were obtained (Fig. 6, upper panel). With respect to DT40 cells and rat ES cells (it is difficult to observe a micronucleation image in the bright field), cells were collected, suspended with 0.075 M KCl at room temperature for 15 minutes, fixed with a Carnoy's fluid, applied onto a glass slide and stained with DAPI. The fixed cells were observed by fluorescence microscopy (Fig. 6, lower panel). The results demonstrate that treatment with paclitaxel (4 nM, 25 nM, 100 nM, 400 nM) with subsequent treatment with reversine (0 nM, 500 nM, 1000 nM, 1500 nM) during the preceding treatment was capable of inducing micronucleation in various types of non-human animal cells.

### <Example 2>

Transfer of artificial chromosome vector from human immortalized mesenchymal stem cell to human cell line HT1080

### [A] Transfer of HAC vector labeled with a drug resistant gene into HT1080

A HAC vector labeled with a drug resistant gene and contained in hiMSC is transferred into human cells HT1080. At this time, a chemical agent treatment condition which allows efficient induction of micronucleation for the human cells was used and the transfer was made into HT1080 by microcell-mediated chromosome transfer.

### [A.1.1] Microcell-mediated chromosome transfer and isolation of drug resistant clone

Donor cells (also referred to as a "donor"), i.e., a HAC vector-comprising hiMSC, were cultured in 12 cell-culture flasks having a bottom area of 25 cm². At a 70% confluence, a micronucleation induction treatment (paclitaxel 1 nM to 1 µM and reversine 1 nM to 1 µM) was carried out at 37°C in the condition of 5% CO₂ for 48 hours. After completion of the micronucleation induction (treatment), the culture medium (medium) in the flasks was removed by aspiration. The flasks were filled up to a level of 90% with cytochalasin B. The flasks were inserted into specialized containers for a large-size high-speed centrifuge (BECKMAN) and hot water (34°C) was added so as not to completely cover the flask. Centrifugation (Rortor ID10.500, 8,000 rpm, 1h, 34°C) was carried out. After completion of centrifugation, cytochalasin B was recovered. The pellets in the flasks were collected with 2 ml of serum-free medium DMEM in a 15-ml tube and slowly filtered by filters (in the order of 8 µm→5 µm→3 µm). The tubes were centrifuged (1,200 rpm, 5 minutes, room temperature (R.T)) and the supernatant was removed by aspiration. The pellets of individual tubes were collected in a 5 ml serum-free medium DMEM, suspended and centrifuged (2000 rpm, 5 minutes). In this manner, microcells were collected. Recipient cells (also referred to as "recipient") HT1080 were seeded in a 6 cm-diameter cell-culture dish on the previous day (in advance) so as to reach a 90% confluence and washed with serum-free medium (DMEM). The micronuclei purified were resuspended with 2 ml of serum-free culture solution containing PHA-P (SIGMA) and gently seeded on HT1080, from which the serum-free culture solution (DMEM) was already removed. The culture dish was allowed to stand still at 37°C in the condition of 5% CO₂ for 20 minutes. The supernatant was removed and fusion was carried out with 1 ml of PEG1000 solution (Wako, Japan) [5 g of PEG1000 is completely dissolved in serum-free DMEM medium and dimethyl sulfoxide (1 mL) is added thereto and sterilized by filtration] for accurately 90 seconds. The fused cells were washed three times with 5 ml of serum-free culture solution (DMEM) and incubated with conventional HT1080 culture solution (5 ml) at 37°C, overnight. Twenty four hours later, the fused cells were re-seeded in 6 culture dishes of 10 cm in diameter. Further, 24 hours later, blasticidin S was added so as to be 8 µg/ml and selective culture was carried out for 3 to 4 weeks. In a single microcell-mediated chromosome transfer operation, three GFP fluorescence positive and drug resistant colonies emerged in total. These colonies were isolated, proliferated and subjected to the following analysis.

### [A.1.2] Verifying the transfer of HAC vector into drug resistant clone by FISH analysis

The clones obtained in the above were subjected to FISH analysis in accordance with the method described in the report by Shinohara et al. (Human Molecular Genetics, 10: 1163-1175, 2001), in which a red probe (arrow) is bound to alphoid sequence and a green probe (arrowhead) is bound to pCX-EGFP vector. As a result of the analysis, the presence of the HAC vector was confirmed in three clones (Fig. 7).

### <Example 3>

Transfer of artificial chromosome vector from human iPS cell to human cell line HT1080

### [A] Transfer of MAC vector labeled with drug resistant gene into HT1080

A MAC vector labeled with a drug resistant gene and contained in a human iPS cell is transferred into human cells, HT1080. At this time, a chemical agent treatment condition which allows efficient induction of micronucleation for the human cells is used and the transfer is made into HT1080 by microcell-mediated chromosome transfer.

### [A.1.1] Microcell-mediated chromosome transfer and isolation of drug resistant clone

Donor human iPS cells were seeded in 12 cell-culture flasks previously coated with 0.5 µg/cm² Laminin-511 and 10 µM Y-27632 was added thereto. Culture was carried out at 37°C in the condition of 5% CO₂ for 24 hours. Thereafter, the medium was exchanged with iPS culture medium conventionally used. Proliferation was carried out up to a 90% confluence. At this time, MAS (0.2 µg/mL) was added and culture was carried out at 37°C in the condition of 5% CO₂ for 48 hours. In this manner, micronuclei were formed. The culture solution was removed. A flask for centrifugation was filled with cytochalasin B (10 µg/ml, Sigma) solution previously kept at 37°C and spun at 34°C and 8000 rpm for one hour. Microcells were suspended in serum-free DMEM medium and purified by filters (8 µm, 5 µm, 3 µm). After purification, the microcells were centrifuged at 2000 rpm for 10 minutes and suspended in serum-free DMEM medium (5 ml). The microcells were suspended in 5 ml of serum-free DMEM medium and purified by filters (8 µm, 5 µm, 3 µm). After purification, centrifugation was carried out at 2000 rpm for 10 minutes. Recipient cells HT1080 were seeded in a 6 cm-diameter cell-culture dish on the previous day (in advance) so as to reach a 90% confluence and washed with serum-free medium (DMEM). The micronuclei purified were resuspended with 2 ml of serum-free culture solution containing PHA-P (SIGMA) and gently seeded on HT1080 from which the serum-free culture solution (DMEM) was already removed. The culture dish was allowed to stand still at 37°C in the condition of 5% CO₂ for 20 minutes. The supernatant was removed and fusion was carried out with 1 ml of PEG1000 solution (Wako, Japan)[5 g of PEG1000 is completely dissolved in serum-free DMEM medium and dimethyl sulfoxide (1 mL) is added thereto and sterilized by filtration] for accurately 90 seconds. The fused cells were washed three times with 5 ml of serum-free culture solution (DMEM) and incubated with conventional HT1080 culture solution (5 ml) at 37°C, overnight. Twenty four hours later, the fused cells were re-seeded in 6 culture dishes of 10 cm in diameter. Further, 24 hours later, G418 was added so as to be 800 µg/ml and selective culture was carried out for 3 to 4 weeks. In a single microcell-mediated chromosome transfer operation, four GFP fluorescence positive and drug resistant colonies emerged in total (Fig. 8). These colonies were isolated, proliferated and subjected to the following analysis.

### [A.1.2] Verifying the transfer of MAC vector into drug resistant clone by FISH analysis

The clones obtained in the above were subjected to FISH analysis in accordance with the method described by Shinohara et al. (Human Molecular Genetics, 10: 1163-1175, 2001), in which a red probe (arrow) was bound to mouse cot-1 sequence, and a green probe (arrowhead) was bound to pCX-EGFP vector. As a result of the analysis, the presence of the MAC vector was verified in four clones (Fig. 9).

### <Example 4>

### Transfer of Chromosome X from human iPS cell (male) to human iPS cell (female)

[A] Using the method described in Example 3, a human internal chromosome is transferred from a human iPS cell to another human iPS cell by microcell-mediated chromosome transfer.

### [A.1.1] Microcell-mediated chromosome transfer and isolation of drug resistant clone

A male-derived human iPS cells (karyotype: 46, XY), which are induced from donor HFL-I-derived (RCB0521, RIKEN BRC) cells by use of Yamanaka factors, are seeded in 12 cell-culture flasks previously coated with 0.5 µg/cm² Laminin-511, and 10 µM Y-27632 is added thereto. Culture is carried out at 37°C in the condition of 5% CO₂ for 24 hours (transferred vector free group). Alternatively, CD46 of the iPS cells is deleted and anti-CD9-ScFv fused MV-H expression plasmid vector (12 µg) and MV-F expression plasmid (12 µg) are transferred by use of Lipofectamine2000 in accordance with the (vector-transferred group). Thereafter, the transferred vector free group and the vector-transferred group are both proliferated in a conventional iPS culture medium after medium exchange. At a 90% confluence, MAS (0.2 µg/mL) is added and culture is carried out at 37°C in the condition of 5% CO₂ for 48 hours to form micronuclei. The culture solution is removed. A flask for centrifugation is filled with cytochalasin B (10 µg/ml, Sigma) solution previously kept at 37°C and spun at 34°C and 8000 rpm for one hour. Microcells are suspended in serum-free DMEM medium and purified by filters (8 µm, 5 µm, 3 µm). After purification, centrifugation is carried out at 2000 rpm for 10 minutes and suspension is carried out in serum-free DMEM medium (5 ml). Microcells are suspended in 5 ml of serum-free DMEM medium and purified by filters (8 µm, 5 µm, 3 µm). After purification, centrifugation was carried out at 2000 rpm for 10 minutes. Recipient human iPS cells (karyotype: 46, XX) in which HPRT gene derived from 201B7 (HPS0063, RIKEN BRC) is deleted or human iPS cells derived from a patient with Lesch-Nyhan syndrome (human iPS cell line induced from fibroblasts derived from a patient with JCRB0072/KURB1995/KURB1996, JCRB or ATCC™ CRL-1110™ and ATCC) are seeded in a 6 cm-diameter cell-culture dish on the previous day (in advance) so as to reach a 90% confluence and washed with serum-free medium (DMEM). In the microcell group in which a vector was not transferred into donor cells, purified micronuclei are resuspended in a serum-free culture solution (2 ml) containing PHA-P (SIGMA) and gently seeded on human iPS cells from which serum-free culture solution (DMEM) is already removed. The culture dish is allowed to stand still at 37°C in the condition of 5% CO₂ for 20 minutes. The supernatant is removed and fusion is carried out with 1 ml of PEG1000 solution (Wako, Japan)[5 g of PEG1000 is completely dissolved in serum-free DMEM medium and dimethyl sulfoxide (1 mL) is added thereto and sterilized by filtration] for accurately 90 seconds. The fused cells are washed three times with 5 ml of serum-free culture solution (DMEM) and incubated with conventional culture solution (5 ml) for human iPS cell at 37°C, overnight. The microcell group having a vector transferred is suspended in human iPS cell culture medium and added to a dish in which recipient cells are cultured and incubated. Twenty four hours later, both the vector-transferred group and the transferred vector free group are re-seeded in six 10-cm diameter culture dishes. Further 24 hours later, addition is made so as to have 1 x HAT and selective culture is carried out for 3 to 4 weeks. Drug resistant colonies are isolated and proliferated and the following analysis is carried out.

### [A.1.2] Karyotype analysis by quinacrine/Hoechst double staining

The HAT resistant clone is subjected to quinacrine/Hoechst double staining. The chromosome image of the clone subjected to quinacrine/Hoechst double staining is observed by fluorescence microscopy. In this manner, it is possible to confirm that an Chromosome X has been further transferred into a cell having a karyotype: 46, XX.

### <Example 5>

Improvement of efficiency of chromosome introduction method by microcell-mediated chromosome transfer through micronucleation induction by treatment of CHO cells with paclitaxel and reversine

### [A] Introduction of HAC vector labeled with a drug resistant gene into HT1080

HAC vector labeled with a drug resistant gene contained in CHO MHG#4a was introduced into human cells, HT1080. At this time, a chemical agent treatment condition which allows efficient induction of micronucleation into human cells is used and introduction is made into HT1080 by microcell-mediated chromosome transfer, and then, the number of drug-resistant colonies emerged are compared.

### [A.1.1] Microcell-mediated chromosome transfer and isolation of drug resistant clone

HAC vector-comprising donor CHO cells were seeded in a culture dish having a diameter of 10 cm and cultured up to a 90% confluence. Into this, a MV-H expression plasmid vector (12 µg) and a MV-F expression plasmid (12 µg) were transferred by use of Lipofectamine 2000 in accordance with the specification. Twenty four hours later, The cells were passed in three 25 cm²-flasks. From the following day, a colcemid treatment (F12/20%FBS/0.1 µg/mL colcemid), as a micronucleation-inducing drug treatment, was carried out for 48 hours. Medium exchange was carried out again and a colcemid treatment (F12/20%FBS/0.1 µg/mL colcemid) was carried out for 24 hours to form micronuclei. Alternatively, paclitaxel and reversine treatment (F12/20%FBS/384 nM paclitaxel/1536 nM paclitaxel) was carried out for 48 hours to form micronuclei. After the micronucleation-inducing drug treatment, the culture medium in the flasks was removed, the flasks were filled up to a level of 90% with cytochalasin B. The flasks were set in specialized centrifuge tubes and hot water (34°C) was added so as not to completely cover the flask. Centrifugation was carried out by a JLA-10, 500Rotor (BECKMAN) at 8,000 rpm, 1 hour, 34°C. After centrifugation, cytochalasin B was collected and removed and the pellets in the flasks were collected with 2 ml of serum-free medium DMEM in a 50-mL tube. Filtration was carried out by filters (in the order of 8 µm→5 µm→3 µm) and centrifugation (2000 rpm, 10 minutes, room temperature (R.T)) was carried out. After completion of centrifugation, the supernatant was removed. The pellets of individual tubes were suspended in recipient cell culture medium, added to a dish in which recipient cells were cultured, and incubated. As the recipient cells, HT1080 cells, which were cultured up to a 90% confluence, were used. Twenty four hours later, reseeding was carried out in five 10 cm-diameter culture dishes. Further, 24 hours later, selective culture was carried out by 3 µg/mL blasticidin S. The selective culture was carried out for 3 to 4 weeks. The number of colonies emerged was counted and shown in a graph (Fig. 10). As a result, the group treated with paclitaxel and reversine showed a HAC vector introduction efficiency, which was 18.3 times as high as that of the colcemid processing group (Fig. 10).

### <Example 6>

Introduction of human Chromosome X from human immortalized mesenchymal stem cell to human cell line HT1080

[A] Using the method described in Example 2, internal Chromosome X was introduced from a human immortalized mesenchymal stem cell into human HT1080. At this time, a chemical agent treatment condition which allows efficient induction of micronucleation into human cells was used and introduction was made into HT1080 by microcell-mediated chromosome transfer.

### [A.1.1] Microcell-mediated chromosome transfer and isolation of drug resistant clone

Donor hiMSC cells were cultured in 12 cell-culture flasks having a bottom area of 25 cm² and previously coated with Poly-L-Lysine (50 µg/mL) for 1 hour. At a 70% confluence, a micronucleation induction treatment with (paclitaxel (1 nM to 1 µM) and reversine (1 nM to 1 µM)) was carried out at 37°C in the condition of 5% CO₂ for 48 hours. After completion of micronucleation induction, the culture medium in the flasks was removed by aspiration. The flasks were filled up to a level of 90% with cytochalasin B. The flasks were inserted into specialized containers for a large-size high-speed centrifuge (BECKMAN) and hot water (34°C) was added so as not to completely cover the flasks. Centrifugation (Rortor ID 10.500, 8,000 rpm, 1 hour, 34°C) was carried out. After completion of centrifugation, cytochalasin B was recovered. The pellets in the flasks were collected with 2 ml of serum-free medium DMEM in a 15-ml tube and slowly filtered by filters (in the order of 8 µm→5 µm→3 µm). Individual tubes were centrifuged (1,200 rpm, 5 minutes, room temperature (R.T)). The supernatants were removed by aspiration, the pellets of the individual tubes are collectively placed in 5-ml serum-free medium DMEM, suspended and centrifuged (2000 rpm, 5 minutes) to recover microcells. Recipient cells (HT1080 HPRT-defective cell line) were seeded in a 6 cm-diameter cell-culture dish on the previous day (in advance) so as to reach a 90% confluence and washed with serum-free medium (DMEM). Purified micronuclei were resuspended in 2 ml of a serum-free culture solution containing PHA-P (SIGMA) and gently seeded on HT1080 from which the serum-free culture solution (DMEM) was already removed. The culture dish was allowed to stand still at 37°C in the condition of 5% CO₂ for 20 minutes. The supernatant was removed and fusion was carried out with 1 ml of PEG1000 (Wako, Japan) solution [5 g of PEG1000 is completely dissolved in serum-free DMEM medium and dimethyl sulfoxide (1 mL) is added thereto and sterilized by filtration] for accurately 90 seconds. The fused cells were washed three times with 5 ml of serum-free culture solution (DMEM) and incubated with HT1080 culture solution (5 ml) ordinarily used at 37°C, overnight. Twenty four hours later, the fused cells were re-seeded in 6 culture dishes of 10 cm in diameter. Further, 24 hours later, 50 × HAT supplement (Sigma) was added so as to be a concentration of 1 × HAT and selective culture was carried out for 3 to 4 weeks. In a single microcell-mediated chromosome transfer operation, three GFP fluorescence positive and drug resistant colonies emerges in total. These colonies were isolated, proliferated and subjected to the following analysis.

### [A.1.2] Verifying the transfer of Chromosome X into drug resistant clone by FISH analysis

The drug resistant clone obtained was subjected to FISH analysis in accordance with the method described in the report by Shinohara et al. (Human Molecular Genetics, 10: 1163-1175, 2001). The following primer was used as a probe for detecting Chromosome X. Chromosome X specific alpha-satellite was amplified by using the following primers and a red probe was prepared and put in use.
DXZ1 ChrX alpha-satellite F: 5'-ATAATTTCCCATAACTAAACACA-3' (SEQ ID NO: 1)
DXZ1 ChrX alpha-satellite R: 5'-TGTGAAGATAAAGGAAAAGGCTT-3' (SEQ ID NO: 2)

A control experiment was carried out using HT1080 HPRT defective cell line into which Chromosome X was not transferred, and an acquired drug resistant clone. Since HT1080 HPRT defective cell line was a male-derived cell, it was shown that a single Chromosome X alone is contained (Fig. 11, left panel). In contrast, the acquired drug resistant clone, it was shown that two Chromosome Xs were contained (Fig. 11, right panel).

### <Example 7>

Introduction of human Chromosome X from human iPS cell (female) to human cell line HT1080 HPRT defective cell line

[A] Using the method described in Example 3, a human internal chromosome was transferred from a human iPS cell into a human cell line, i.e. HT1080 HPRT defective cell line, by microcell-mediated chromosome transfer.

### [A.1.1] Microcell-mediated chromosome transfer and isolation of drug resistant clone

Donor female-derived human iPS cell line 201B7 (RCB, HPS0063) (karyotype: 46, XX) was seeded in 12 cell-culture flasks previously coated with 0.5 µg/cm² Laminin-511 and 10 µM Y-27632 was added thereto. Culture was carried out at 37°C in the condition of 5% CO₂ for 24 hours. Thereafter, the medium was exchanged with iPS culture medium conventionally used and proliferation was carried out. At a 90% confluence, culture was carried out in the conditions of paclitaxel (20 nM), reversine (500 nM), 37°C, 5% CO₂ for 48 hours to form micronuclei. The culture solution was removed. A flask for centrifugation was filled with cytochalasin B (10 µg/ml, Sigma) solution previously kept at 37°C and spun at 34°C and 8000 rpm for one hour. Microcells were suspended in serum-free DMEM medium and purified by filters (8 µm, 5 µm, and 3 µm). After purification, centrifugation was carried out at 2000 rpm for 10 minutes and suspension was carried out in serum-free DMEM medium (5 ml). Microcells were suspended in 5 ml of serum-free DMEM medium and purified by filters (8 µm, 5 µm, and 3 µm). After purification, centrifugation was carried out at 2000 rpm for 10 minutes. Recipient cells, i.e. 6-thioguanine resistant HT1080 cells that are defective in HPRT gene, are seeded in 6-cm diameter cell-culture dishes on the previous day (in advance) so as to reach a 90% confluence and washed with serum-free medium (DMEM). Purified micronuclei are resuspended in serum-free culture solution (2 ml) containing PHA-P (SIGMA) and gently seeded on human iPS cells, from which serum-free culture solution (DMEM) was already removed. The culture dishes were allowed to stand still at 37°C in the condition of 5% CO₂ for 20 minutes. The supernatant was removed, and fusion was carried out with 1 ml of PEG1000 solution (Wako, Japan) [5 g of PEG1000 is completely dissolved in serum-free DMEM medium and dimethyl sulfoxide (1 mL) is added thereto and sterilized by filtration] for accurately 90 seconds. The fused cells were washed three times with 5 ml serum-free culture solution (DMEM) and incubated with conventional culture solution (5 ml) at 37°C, overnight. Twenty four hours later, the fused cells were re-seeded in six 10-cm diameter culture dishes. Further 24 hours later, HAT was added so as to be 1 x HAT and selective culture was carried out for 3 to 4 weeks. A drug resistant colony was isolated, proliferated and subjected to the following analysis.

### [A.1.2] Multicolor FISH analysis

The HAT resistant clone was subjected to multicolor FISH analysis. Images of chromosomes stained with a multicolor FISH reagent (MetaSystems) were observed by fluorescence microscopy. As a result, the clone obtained was a tetraploid but had three Chromosome Xs, though HT1080 normal karyotype contains a single Chromosome X in the case of a diploid, and two Chromosome Xs in the case of a tetraploid. From the result, it could be confirmed that a foreign Chromosome X had been transferred (Fig. 12).

### [B.1.1] Foreign Chromosome X-transferred cell line, HAT and 6-thioguanine-sensitivity evaluation test

HAT resistant cells obtained (1 × 10⁴ cells) were seeded in a well of a 6-well plate. From the following day, the cells were treated with 6-thioguanine (6TG) (20 µM in concentration) for 120 hours to evaluate resistance to 6-thioguanine. Since HT1080 wild type cell line has a normal HPRT gene on Chromosome X, the cell line is resistant to HAT and thus metabolized 6-thioguanine and underwent cytotoxicity (Fig. 13, left panel). Since HT1080 HPRT defective cell line lacks (internal) HPRT gene in an (internal) Chromosome X, cytotoxicity with 6-thioguanine was not shown. HT1080 HPRT defective cell line was insensitive to 6-thioguanine. In contrast, cell proliferation was stopped by treatment with 1 × HAT, thereby indicating HAT sensitivity (Fig. 13, middle panel). In a foreign Chromosome X-comprising HT1080 HPRT deficient cell line, which was obtained by transferring a foreign normal Chromosome X into the HT1080 HPRT defective cell line, cytotoxicity with 6-thioguanine was observed similarly to HT1080 wild type cell line; however, the cell line was HAT resistant (Fig. 13, right panel). These demonstrate that human iPS cell-derived Chromosome X having a normal cell HPRT gene was successfully transferred from a human iPS cell into HT1080 cell.

### INDUSTRIAL APPLICABILITY

The present invention provides a technique for directly introducing DNA of interest from a human cell or non-human animal cell (for example, rodent cell) (as a donor cell) comprising the DNA of interest to a human cell or non-human animal cell (as a recipient cell), and thus, the invention being useful for regenerative medicine and drug development using, for example, a human cell such as a human iPS cell.

### SEQUENCE LISTING FREE TEXT

SEQ ID NO: 1: primer
SEQ ID NO: 2: primer

All publications, patents and patent applications cited herein are incorporated herein by reference in their entirety.

## Claims

1. A method for preparing human cell-derived microcells from human cells (excepting cancer cells) comprising DNA of interest, comprising culturing the human cells in a medium containing at least one micronucleus inducer selected from the group consisting of microtubule polymerization inhibitors (excepting colcemid), microtubule depolymerization inhibitors, and spindle checkpoint inhibitors, thereby to produce human cell-derived microcells, and collecting the human cell-derived microcells comprising the DNA of interest.

2. The method according to claim 1, wherein the DNA of interest is a normal human chromosome.

3. The method according to claim 1, wherein the DNA of interest is a human artificial chromosome or mammalian artificial chromosome comprising a gene of interest.

4. The method according to any one of claims 1 to 3, wherein the microtubule polymerization inhibitor is a mixture of colchicine and vincristine.

5. The method according to any one of claims 1 to 3, wherein the microtubule depolymerization inhibitor is paclitaxel, docetaxel, or a mixture of paclitaxel and docetaxel.

6. The method according to any one of claims 1 to 3, wherein the spindle checkpoint inhibitor is reversine, hesperadin, or a mixture of reversine and hesperadin.

7. A method for preparing non-human animal cell-derived microcells from non-human animal cells (excepting cancer cells) comprising DNA of interest, comprising culturing the non-human animal cells in a medium containing at least one micronucleus inducer selected from the group consisting of reversine, paclitaxel, or a mixture of reversine and paclitaxel, and a mixture of colchicine and vincristine, thereby to produce non-human animal cell-derived microcells, and collecting the non-human animal cell-derived microcells comprising the DNA of interest.

8. The method according to claim 7, wherein the non-human animal cell is a rodent cell.

9. The method according to claim 8, wherein the rodent cell is a Chinese hamster ovary (CHO) cell or a mouse A9 cell.

10. The method according to any one of claims 7 to 9, wherein the DNA of interest is a normal human chromosome.

11. The method according to any one of claims 7 to 9, wherein the DNA of interest is a human artificial chromosome or a mammalian artificial chromosome, which comprises a gene of interest.

12. A method for preparing human cells or non-human animal cells comprising DNA of interest, comprising: a step of preparing human cell-derived microcells comprising DNA of interest by the method according to any one of claims 1 to 6, or a step of preparing non-human animal cell-derived microcells comprising DNA of interest by the method according to any one of claims 7 to 11; a step of fusing the human cell-derived microcells or the non-human animal cell-derived microcells, which serve as donor cell, with human cells or non-human animal cells, respectively, serving as recipient cell; and a step of collecting the human cells or non-human animal cells comprising the DNA of interest.

13. The method according to claim 12, wherein the non-human animal cell is a rodent cell.

14. The method according to claim 13, wherein the rodent cell is a Chinese hamster ovary (CHO) cell or a mouse A9 cell.

15. The method according to claim 12, wherein the DNA of interest is a normal human chromosome.

16. The method according to claim 12, wherein the DNA of interest is a human artificial chromosome or mammalian artificial chromosome comprising a gene of interest.


SEQUENCE LISTING

<110>  NATIONAL UNIVERSITY CORPORATION TOTTORI UNIVERSITY
       Trans Chromosomics, Inc.

<120>  Method of Producing Animal Cells Comprising DNA of Interest
       By Microcell-Mediated Chromosome Transfer

<130>  PH-8045-PCT

<150>  JP 2018-191994
<151>  2018-10-10

<160>  2

<170>  PatentIn version 3.5

<210>  1
<211>  23
<212>  DNA
<213>  Artificial

<220>
<223>  primer

<400>  1
ataatttccc ataactaaac aca                                              23


<210>  2
<211>  23
<212>  DNA
<213>  Artificial

<220>
<223>  primer

<400>  2
tgtgaagata aaggaaaagg ctt                                              23